# EUROPEAN PATENT APPLICATION

(11) **EP 1 970 057 A1**
(43) Date of publication of application: **17.09.2008**
(21) Application number: 07103985.3
(22) Date of filing: 13.03.2007
(51) Int. Cl.: A61K 9/51, A61K 31/5377, C07D 413/06

(54) **Process for the preparation of stable pharmaceutical compositions of 2-(R) - (1-(R)-(3, 5- bis (trifluoromethyl) phenyl) ethoxy)-3-(S)-(4-fluoro) phenyl-4-(3-(5-oxo-1H, 4H-1, 2, 4-triazolo) methylmorpholine (aprepitant)**

(71) Applicant: SANDOZ AG, 4056 Basel (CH)
(72) Inventor: The designation of the inventor has not yet been filed
(74) Representative: Kröger, Bernd

(57) **Abstract**

The present invention relates to a process for the preparation of a stable pharmaceutical composition of 2-(R) - (1-(R)-(3,5- bis (trifluoromcthyl)phcnyl) ethoxy)-3-(S)-(4-fluoro)phenyl-4-(3-(5-oxo-1H,4H-1,2,4-triazolo) methylmorpholine (aprepitant), with a view to increase its solubility and bioavailability.

## Description

### Field of the Invention

The present invention relates to stable pharmaceutical compositions of 2-(R)-(1-(R)-(3, 5-bis(trifluoromethyl)phenyl)ethoxy)-3-(S)-(4-fluoro) phenyl-4-(3-(5-oxo-1H, 4H-1, 2, 4-triazolo) methylmorpholine and methods for their preparation.

### Background of the Invention

2-(R)-(1-(R)-(3,5-bis(trifluoromethyl)phenyl)ethoxy)-3-(S)-(4-fluoro) phenyl-4-(3-(5-oxo-1H, 4H-1, 2, 4-triazolo) methylmorpholine (aprepitant) is an antagonist of Substance P, which is a naturally occurring undecapeptide belonging to the tachykinin family of peptides. There is evidence for the use of a tachykinin receptor antagonist in the treatment of various diseases or disorders. Aprepitant is poorly water soluble and thus exhibits slow oral absorption, resulting in very low oral bioavailability.

Bioavailability is defined as the degree to which a drug becomes available to the target tissue after administration. The absorption of the drug in the body is dependent on the bioavailability of the drug. To facilitate absorption, the drug must be in soluble form at the site of absorption.

Poorly water-soluble drugs are those drugs that have solubility less than about 10 mg/ml. Poorly soluble drugs have low bioavailability and thus pose a problem for the formulation scientist. Various techniques are employed to increase the solubility of the drug which include, but are not restricted to, decreasing the particle size, complexation ,formation of solid solution, changing the surface characteristics of the particles and incorporation of drug particles into colloidal systems like nanoparticles and liposomes. Ford J.L. in the review article (The status of solid dispersions in Pharm. Acta Helv. 61 (3)1986,69-88) has described the advantages and techniques to improve the solubility of poorly water soluble drugs by formation of solid dispersions.

The rate of dissolution of a drug is inversely proportional to the particle size of the drug. Consequently, methods of making finely-divided drug have been studied and efforts have been made to control the size of drug particles in pharmaceutical compositions. Techniques such as dry milling, wet grinding and commercial airjet milling have provided particles with an average particle size ranging from as low as about 1µm to about 50µm (1,000-50,000 nm)

European Patent 499,299 describes a technique for preparing pharmaceutical compositions comprising loading drugs into liposomes or polymers. Such techniques have problems and limitations. For example, a lipid soluble drug is often required for preparing suitable liposomes. Further, unacceptably large amounts of the liposome or polymer are often required to prepare unit drug doses. Furthermore, techniques for preparing such pharmaceutical compositions tend to be complex. A principal technical difficulty encountered with emulsion polymerization is the removal of contaminants, such as unreacted monomer or initiator, which can be toxic, at the end of the manufacturing process.

U. S. Patent 4,540,602 (the '602 patent) discloses a method for the preparation of an activated pharmaceutical composition containing a solid drug. Sparingly soluble drug is dispersed in water, in the presence of a water-soluble high-molecular weight substance to form a disperse system containing the drug in the form of finely divided particles substantially not greater than 10µm in diameter. The dispersion medium is then removed from the disperse system, whereby a pharmaceutical composition containing finely divided drug coated with the water-soluble high-molecular substance is obtained. The '602 patent teaches various methods for dispersing the drug in water to form a disperse system containing the drug in the form of finely divided particles. Pulverization of drug in water is one of the techniques.

European Patent 275,796 (the '796 patent) describes the production of colloidally dispersible systems comprising a substance in the form of spherical particles smaller than 500 nm. This method involves a precipitation effected by mixing a solution of the substance and a miscible non-solvent for the substance, and results in the formation of non-crystalline nanoparticles. According to the '796 patent, precipitation techniques for preparing particles tend to provide particles contaminated with solvents. Such solvents are often toxic and it can be very difficult, if not impossible, to reduce the solvent content to pharmaceutically acceptable levels to be practical.

U.S. patent 5,145,684 describes stable, dispersible drug nanoparticles and a method for preparing such particles by wet milling in the presence of grinding media in conjunction with a surface modifier. The particles can be formulated into pharmaceutical compositions exhibiting remarkably high bioavailability

U.S. Patent Application 2004/0214746 describes processes involving nanoparticulate technology to increase solubility and improve bioavailability of less soluble drugs such as 2-(R) - (1-(R)-(3,5- bis (trifluoromethyl)phenyl) ethoxy)-3-(S)-(4-fluoro)phenyl-4-(3-(5-oxo-1H,4H-1,2,4-triazolo) methylmorpholine (aprepitant). The process described in this application includes wet grinding as one of the techniques to increase solubility.

PCT patent application 2007/016582 describes co precipitates comprising of amorphous aprepitant and pharmaceutically acceptable carriers.

Most of the prior art techniques described suffer from disadvantages during scale-up and therefore, manufacturing on a large scale is challenging.

It would, therefore, be desirable to provide stable pharmaceutical compositions of poorly soluble drugs which can be readily prepared and that show improved solubility and, in turn, good bioavailability over the drug substance.

### Summary of the Invention

The present invention relates to a process for the preparation of a stable pharmaceutical composition of 2-(R)-(1-(R)-(3,5-bis(trifluoromethyl)phenyl)ethoxy)-3-(S)-(4-fluoro)phenyl-4-(3-(5-oxo-1H,4H-1,2,4-triazolo) methylmorpholine (aprepitant). Specifically, the present invention relates to a process for the preparation of a solid solution of aprepitant.

### Brief description of drawing:

Figure I: X-Ray Diffraction of aprepitant solid solution.

### Description of the Invention

The present invention relates to a stable pharmaceutical composition of aprepitant and a process for preparing the same, with a view to increase its solubility and bioavailability.

The present invention provides a process for the preparation of a stable pharmaceutical composition of aprepitant comprising:
(a) dissolving aprepitant or a pharmaceutically acceptable salt thereof with at least one polymer in a suitable solvent to form a solution;
(b) spraying the solution onto solid carrier spheres; and
(c) drying the solid carrier spheres to remove the solvent.

The present invention relates to a process for the preparation of a solid solution formulation of aprepitant and a process for its preparation.

In accordance with the present invention, "solid solution" means a solid-state system containing at least two components wherein the first component is dispersed rather uniformly in the second component. The dispersed materials maintain the system in chemically or physically uniform or homogeneous state, or consist of one phase as defined in thermodynamics. The solid solution contains materials in super-homogeneous state such as glassy solid solution as well as in less homogeneous state.

'Organic solvent" as used herein includes, but is not limited to, lower alcohols, chlorinated solvents (1, 2-dichloromethane, chloroform) or any other pharmaceutically acceptable solvents and mixtures thereof.

Formation of solid solution does not result in the formation of a covalent bond and the drug does not form a lattice structure, which would result in crystal formation, instead it results in the formation of a solid solution.

In a solid solution, particle size reduction of the drug within the matrix is achieved to the minimum level; *i.e* the molecular state is achieved. As the carrier dissolves, the drug present in the molecular form leads to the formation of supersaturated solution. This leads to enhancement of the dissolution rate of the poorly soluble drug and results in an increase in bioavailability.

As the solid solutions are exposed to water or gastro-intestinal juices, the water-soluble carrier is released to the internal aqueous solution. Simultaneously, components of the solid dispersions dissolve into minute particles, which increase the surface area of the drug. At this time, the drug particles become smaller and the carrier dissolves completely in a very short time, so that the solubilization of drug is achieved by the carrier in a diffusion layer, which is a minute environment around the drug particles at the early stage of dissolution. Therefore, it is understood that the above-mentioned factors work collectively to increase the solubility and initial dissolution rate of drug.

The selection of the polymer is very important in the formation of solid solutions. The polymer must increase the dissolution rate and must be pharmacologically in-active and non-toxic. Optimum pairing of the drug with the polymer is essential for the formation of a stable solid solution.

Polymers used herein include, but are not limited to water soluble polymers, such as polyethylene glycol(macrogols), polyvinylpyrrolidone, hydroxy propyl methyl cellulose, hydroxyl propyl cellulose, hydroxyethyl cellulose, sorbitol, mannitol and saturated polyglycolized glycerides

In an embodiment of the present invention the polymer used for the formation of solid solution is hydroxy propyl methyl cellulose.

In an embodiment of the present invention the polymer used in the range of 7.5% to 25%.

Solid carriers or solid carrier spheres used herein include, but are not limited to microcrystalline cellulose spheres, sugar starch spheres and lactose spheres.

In the present invention solid solutions are formed when the drug and the polymer solution are sprayed onto solid carrier spheres.

The formation of a solid solution can be analyzed using thermal analysis, X- ray diffraction, microscopic, spectroscopic or thermodynamic techniques. In another aspect of the present invention, X- ray diffraction studies of a solid solution reveal absence of crystalline peaks of aprepitant, thus indicating that aprepitant exists in amorphous form or as a solid solution within the carrier matrix.

The present invention provides a method for the formation of solid solutions of aprepitant without the formation of crystalline lattices of the drug. The solid solution results in the formation of amorphous aprepitant, thus increasing the solubility and, in turn, the bioavailability.

The following example further illustrates the invention.

### Examples:

### Example 1

| Sr. No. | Name of Excipient | Mg/Capsule |
|---|---|---|
| 1 | Aprepitant | 125.000 |
| 2 | Hydroxypropylmethyl cellulose (E-15) | 125.000 |
| 3 | Propylene glycol | 12.500 |
| 4 | Microcrystalline cellulose pellets | 400.000 |

Process:
1. Aprepitant was dissolved in a mixture of solvents.
2. Hydroxypropyl methyl cellulose was dissolved in the same mixture till a clear solution is obtained.
3. Propylene glycol was added to the above mixture and mixed for 10 mins.
4. The above solution was filtered through 100 mesh.
5. The above solution was layered onto the microcrystalline pellets using Fluid bed coater and dried further.
6. Drug loaded pellets were filled into hard gelatin capsules.

### Example 2

| Sr. No. | Name of Excipient | Mg/Capsule |
|---|---|---|
| 1 | Aprepitant | 125.000 |
| 2 | Hydroxypropylmethyl cellulose (E-6) | 125.000 |
| 3 | Propylene glycol | 12.500 |
| 4 | Microcrystalline cellulose pellets | 400.000 |

Process:
1. Aprepitant was dissolved in a mixture of solvents.
2. Hydroxypropyl methyl cellulose was dissolved in the same mixture till a clear solution is obtained.
3. Propylene glycol was added to the above mixture and mixed for 10 mins.
4. The above solution was filtered through 100 mesh.
5. The above solution was layered onto the microcrystalline pellets using Fluid bed coater and dried further.
6. Drug loaded pellets were filled into hard gelatin capsules

### Example 3

| Sr. No. | Name of Excipient | Mg/Capsule |
|---|---|---|
| 1 | Aprepitant | 125.000 |
| 2 | Hydroxypropylmethyl cellulose (E-6) | 93.750 |
| 3 | Propylene glycol | 9.375 |
| 4 | Microcrystalline cellulose pellets | 400.000 |

Process:
1. Aprepitant was dissolved in a mixture of solvents.
2. Hydroxypropyl methyl cellulose was dissolved in the same mixture till a clear solution is obtained.
3. Propylene glycol was added to the above mixture and mixed for 10 mins.
4. The above solution was filtered through 100 mesh.
5. The above solution was layered onto the microcrystalline pellets using Fluid bed coater and dried further.
6. Drug loaded pellets were filled into hard gelatin capsules

### Example 4

| Sr. No. | Name of Excipient | Mg/Capsule |
|---|---|---|
| 1 | Aprepitant | 125.000 |
| 2 | Hydroxypropylmethyl cellulose (E-6) | 125.000 |

1. Aprepitant was dissolved in a mixture of solvents.
2. Hydroxypropyl methyl cellulose was dissolved in the same mixture till a clear solution is obtained.
3. The solvent was evaporated and X-ray diffraction studies were performed. [Refer figure I]

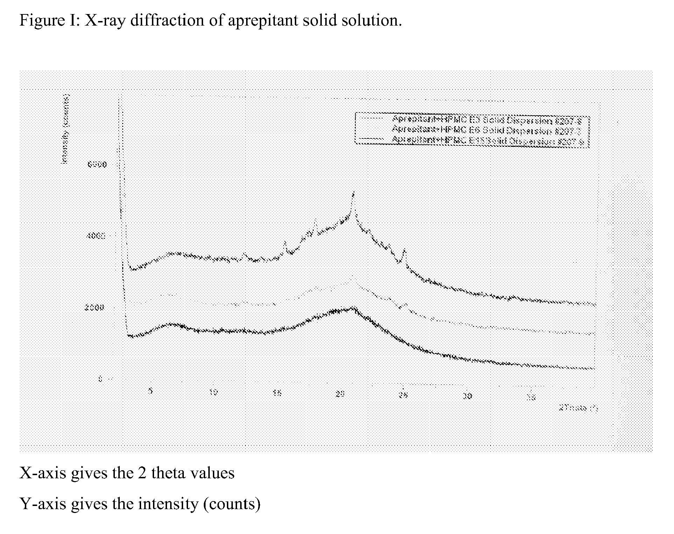

## Claims

1. A stable pharmaceutical composition comprising aprepitant or a pharmaceutically acceptable salt, a polymer and solid carrier spheres.

2. The pharmaceutical composition of claim 1 wherein said composition is a solid solution.

3. The pharmaceutical composition of claim 2 wherein said polymer is hydroxylpropylmethyl cellulose, polyethylene glycol, polyvinylpyrrolidone, hydroxyl propyl cellulose or hydroxyethyl cellulose.

4. The pharmaceutical composition of claim 3 wherein said polymer is hydroxylpropylmethyl cellulose.

5. The pharmaceutical composition of claim 4 wherein the concentration of said hydroxylpropylmethyl cellulose is in the range of 7.5 % to 20%.

6. The pharmaceutical composition of claim 1 wherein said solid carrier spheres are microcrystalline cellulose spheres, sugar starch spheres or lactose spheres.

7. A process for the preparation of a pharmaceutical composition comprising aprepitant, or a pharmaceutically acceptable salt thereof, comprising:
(a) dissolving aprepitant or a pharmaceutically acceptable salt thereof with at least one polymer in a suitable solvent to form a solution;
(b) spraying the solution onto solid carrier spheres; and
(c) drying the solid carrier spheres to remove the solvent.

8. The process of claim 7 wherein said polymer of step (a) is hydroxylpropylmethyl cellulose, polyethylene glycol, polyvinylpyrrolidone, hydroxylpropyl cellulose or hydroxyethyl cellulose

9. The process of claim 8 wherein said polymer is hydroxypropylmethyl cellulose.

10. The process of claim 9 wherein the concentration of said hydroxypropylmethyl cellulose is in the range of 7.5% to 20%.

11. The process of claim 7 wherein said solid carrier spheres of step (b) are microcrystalline cellulose spheres, sugar starch spheres or lactose spheres.

12. The process of claim 11 wherein said solid carrier spheres are microcrystalline cellulose spheres.
